(19) Europäisches Patentamt
European Patent Office
Office européen des brevets

(11) Publication number: **0 258 009 B1**

(12) **EUROPEAN PATENT SPECIFICATION**

(45) Date of publication of patent specification: **11.11.92**

(51) Int. Cl.⁵: **C07D 213/30**

(21) Application number: **87307408.2**

(22) Date of filing: **21.08.87**

# (54) 2,6-Bis(chloromethyloxycarbonyloxymethyl)pyridine.

(30) Priority: **22.08.86 HU 365486**

(43) Date of publication of application:
**02.03.88 Bulletin 88/09**

(45) Publication of the grant of the patent:
**11.11.92 Bulletin 92/46**

(84) Designated Contracting States:
**AT BE CH DE ES FR GB GR IT LI NL SE**

(56) References cited:
**EP-A- 0 233 571**
**CH-A- 484 134**

**CHEMICAL ABSTRACTS, vol. 76, no. 13, 27th March 1972, page 366, no. 72344g, Columbus, Ohio, US; H. BOEHME et al.: "Chloromethyl, mercaptomethyl, and imidomethyl carbonates"**

(73) Proprietor: **Richter Gedeon Vegyészeti Gyár R.T.**
**Gyömröi ut 19-21**
**H-1475 Budapest X(HU)**

(72) Inventor: **T th, J zsef**
**Als -Törökvész u. 10**
**H-1022 Budapest(HU)**

Inventor: **Kovatsits, Máté**
**Kilát u. 19-21**
**H-2016 Leányfalu(HU)**
Inventor: **Csutorás, Lászl**
**Hikádé A.u. 9/a**
**H-1195 Budapest(HU)**
Inventor: **Szab , Gábor**
**Baross u. 96**
**H-1174 Budapest(HU)**
Inventor: **Görög, Sándor**
**Vajda Péter u. 43/b**
**H-1089 Budapest(HU)**
Inventor: **Trischler, Ferenc**
**Uttörö u. 16**
**H-1171 Budapest(HU)**
Inventor: **Holly, Sándor**
**Városház u. 14**
**H-1052 Budapest(HU)**
Inventor: **Francsics, Erzsebet, nee Czinege**
**Elöd u. 6**
**H-1105 Budapest(HU)**
Inventor: **Losonczi, Béla**
**Vércse u. 6**
**H-1124 Budapest(HU)**
Inventor: **Havasi, Gábor**
**Sziklai S. u. 60**
**H-2510 Dorog(HU)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

(74) Representative: **Pett, Christopher Phineas et al**
**Frank B. Dehn & Co. European Patent Attorneys Imperial House 15-19 Kingsway London WC2B 6UZ(GB)**

## Description

This invention relates to the compound 2,6-bis(chloromethyloxy-carbonyloxymethyl)pyridine of formula (I)

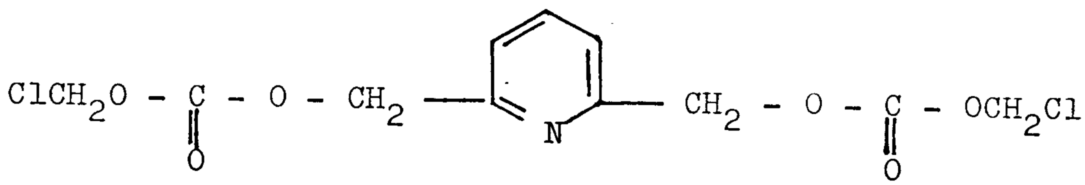

(I)

and to a process for the preparation of this compound.

The compound of formula (I) is an important intermediate for the preparation of 2,6-bis(hydroxymethyl)-pyridine bis(N-methylcarbamate) (generic name: pyridinolcarbamate). Pyridinolcarbamate is a known drug with a useful anti-atherosclerotic and anti-inflammatory action. EP-A-233571 gives one process for its preparation, and CH-A-484134 discloses the preparation of some other 2,6-bis(hydroxymethyl) pyridine derivatives. However, pyridinolcarbamate can also be prepared from the compound of formula (I) by reaction with methylamine by a process e.g. as described below in Example 2. The presence of an alcoholic, e.g. methanolic solvent and the use of lower temperatures, such as below 10 ° C are preferred.

The novel compound of formula (I) is also a useful intermediate for the preparation of other drugs, particularly of carbamate esters.

Some chloromethyl carbonate derivatives are described in Chemical Abstracts 76 (1972), 72344g.

According to one aspect of the invention, there is provided a compound of formula 2,6-bis-(chloromethyloxy-carbonyloxy-methyl)pyridine of formula I

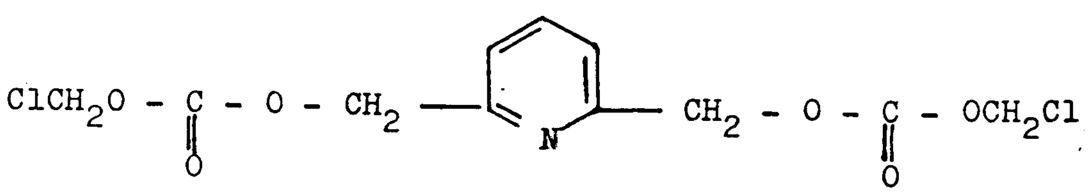

.(I)

According to a further aspect of the invention, there is provided a process for the preparation of the compound of formula (I), which comprises reacting 2,6-bis(hydroxymethyl)pyridine of formula (II)

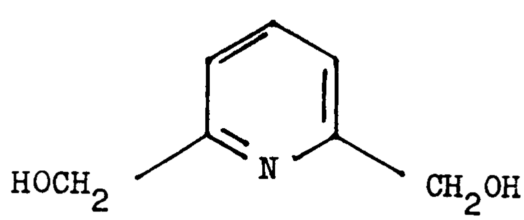

(II)

with chloromethyl chloroformate, optionally in the presence of a further base.

The reaction between the compound of formula II and chloromethyl chloroformate is preferably effected in a polar, aprotic, water-immiscible organic solvent, and at a temperature below 10 ° C.

2,6-Bis(hydroxymethyl)pyridine of the formula (II) may be prepared according to the literature, as described e.g. in the Japanese patent specification No. 68-14,222. Chloromethyl chloroformate may be prepared as described e.g. in the published German patent application No. 3,241,568.

Examples of suitable solvents include: acetonitrile; dimethylformamide; dioxan; tetrahydrofuran; ethyl acetate; ethers; and aromatic hydrocarbons such as benzene or toluene. Halogenated hydrocarbons such as chloroform or dichloromethane are preferred.

Examples of suitable bases include organic bases such as: triethylamine; trimethylamine; N-methylmorpholine and pyridine. The use of pyridine is preferred.

The reaction of the compound of the formula (II) with chloromethyl chloroformate is preferably carried out at a temperature of between -30 °C and +10 °C, most preferably at about -5 °C.

The molar ratio of the compound of formula II: chloromethyl chloroformate: base used is preferably within the range 1:(2.1 to 2.6):(2:1 to 2.6).

One preferred form of the process will now be described in detail:

The chloromethyl chloroformate is dissolved in the desired solvent, preferably in chloroform, and a solution containing an organic base, preferably pyridine, is added at a temperature below 10 °C, preferably at about -5 °C, whilst stirring. The suspension obtained is stirred for about 20 minutes while care is taken to avoid any increase in the temperature. 2,6-Bis(hydroxymethyl)pyridine of the formula (II) is then added to the above mixture at the same temperature at such a rate that the temperature reaches at most -5 °C. The suspension is stirred for about 20 minutes to give a brown solution. After washing this solution with water and evaporating off the solvent, a practically pure product with 95 to 97% of 2,6-bis(chloromethyloxycarbonyloxymethyl)pyridine content is obtained. The product obtained after washing with water and removing the solvent can be used without any purification or, if desired, may be further purified by chromatography on a silica gel column or other adsorbent, e.g. on a synthetic resin.

The physical constants of the product may be determined by using gas chromatography on a 3% OV-210 column at 210 °C.

The following Examples illustrate the invention further:

## Example 1

### Preparation of 2,6-bis(chloromethyloxy-carbonyloxymethyl)pyridine

To a solution containing 13.0 g (0.1 mole) of chloromethyl chloroformate in 40 ml of chloroform, 8 ml (0.1 mole) of pyridine dissolved in 40 ml of chloroform are added at -5 °C at such a rate that the temperature is maintained at -5 °C or below. A suspension is formed which gradually thickens. When all the pyridine has been added, the reaction mixture is stirred at about -5 °C for 15 minutes, then 5.6 g (0.04 mole) of 2,6-bis(hydroxymethyl)pyridine are added to the mixture at the same temperature in several portions at such a rate that the temperature does not increase. During the addition, the mixture becomes thinner and after stirring for about 20 minutes, a brown solution is obtained. 120 ml of water are then added to the mixture under vigorous stirring. The aqueous phase is separated from the organic phase and extracted twice with 40 ml of chloroform each. The cholorformic phases are combined, washed with 20 ml of water and dried over anhydrous magnesium sulphate. After filtration, the solvent is evaporated off and any remaining solvent is removed under vacuum. Thus, 12.2 g of a honey-like oil with a characteristic mildly sharp odour are obtained which contains more than 96% of 2,6-bis-(chloromethyloxycarbonyloxymethyl)pyridine according to the chromatographic measurements. The yield is 98% as calculated for 2,6-bis(hydroxymethyl)pyridine.

Analysis :

Cl calculated at 21.39%
Cl found 21.19%

| | |
|---|---|
| IR ($cm^{-1}$): | C=O 1765, C-O-C 1250; C-Cl 723; aromatic skeleton 1600, 1582; Ar-H def. 790. |
| $^1$H-NMR ($CDCl_3$ δ, ppm): | 5.3 (s, Ar-$CH_2$-O) 5.74 (s, Cl-$CH_2$-O); 7.2-7.9 (m, Ar-H). |

## Example 2

### Preparation of 2,6-bis(hydroxymethyl)pyridine bis(N-methylcarbamate)

3.15 ml of methanolic methylamine solution (containing 33.6% by weight, i.e. 0.024 moles, of methylamine) are added to a solution of 1.3 g (0.004 moles) of 2,6-bis(chloromethyloxy-carbonyloxymethyl)-pyridine (prepared as described in Example 1) at about 0 °C whilst cooling at such a rate that the temperature is maintained at 10 °C or lower. A crystalline product precipitates out. 5 ml of water are then added to the mixture which is then cooled to 2 to 5 °C. The precipitate is separated by filtration and dried to give 0.97 g of the title compound, m.p.: 132-135 °C. The yield is 97% as calculated for 2,6-bis-

(hydroxymethyl)-pyridine. The 2,6-bis(hydroxymethyl)pyridine bis(N-methylcarbamate) content of the product is 98% based on the spectrophotometric determination.

## Claims

### Claims for the following Contracting States : BE, CH, DE, FR, GB, GR, IT, LI, NL, SE

1. 2,6-bis(chloromethyloxy-carbonyloxy-methyl)-pyridlne of the formula (I)

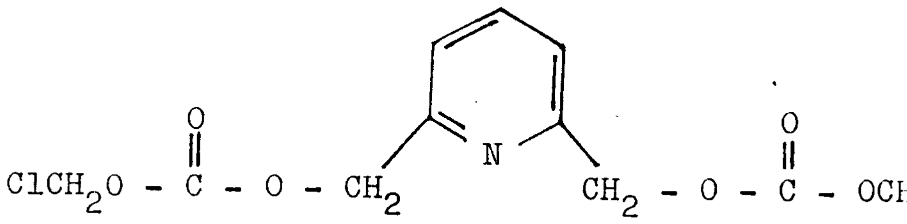

(I)

2. A process for the preparation of 2,6-bis(chloromethyloxy-carbonyloxy-methyl)pyridine of the formula (I)

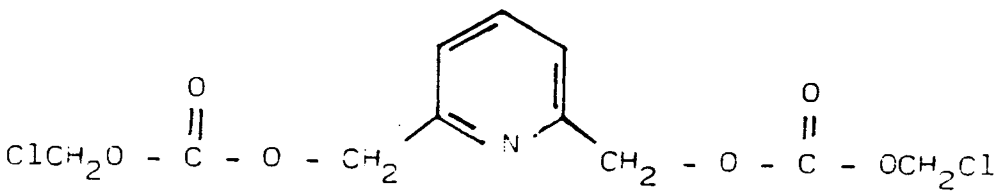

(I)

which comprises reacting 2,6-bis(hydroxymethyl)-pyridine of the formula (II)

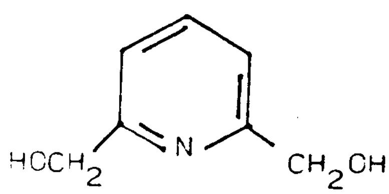

(II)

with chloromethyl chloroformate, optionally in the presence of a further base.

3. A process as claimed in claim 2 wherein the reaction is effected in a polar, aprotic, water-immiscible organic solvent.

4. A process as claimed in claim 2 or claim 3 which comprises using a halogenated hydrocarbon as solvent.

5. A process as claimed in any one of claims 2 to 4 wherein the molar ratio of the compound of formula II: chloromethyl chloroformate: base used is 1:(2.1 to 2.6):(2.1 to 2.6).

6. A process as claimed in any one of claims 2 to 5 which comprises using pyridine as the base.

7. A process as claimed in any one of claims 2 to 6 which comprises carrying out the reaction at a temperature of between -30 ° C and +10 ° C.

8. A process as claimed in any one of claims 2 to 7 which comprises carrying out the reaction at about -5°C.

9. The use of the compound of formula (I) as claimed in claim 1 in the preparation of 2,6-bis-(hydroxymethyl)pyridine carbamate esters.

10. A process for the preparation of 2,6-bis(hydroxymethyl)pyridine bis(N-methylcarbamate) which comprises reacting the compound of formula (I) as defined in claim 1 with methylamine.

**Claims for the following Contracting States : AT, ES**

1. A process for the preparation of 2,6-bis(chloromethyloxy-carbonyloxy-methyl)pyridine of the formula (I)

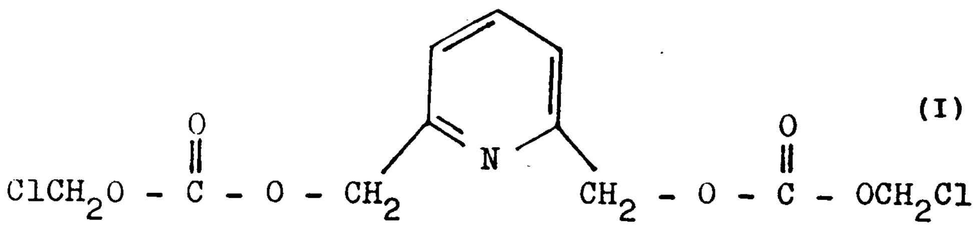

(I)

which comprises reacting 2,6-bis(hydroxymethyl)-pyridine of the formula (II)

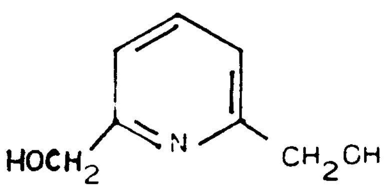

(II)

with chloromethyl chloroformate, optionally in the presence of a further base.

2. A process as claimed in claim 1 wherein the reaction is effected in a polar, aprotic, water-immiscible organic solvent.

3. A process as claimed in claim 1 or claim 2 which comprises using a halogenated hydrocarbon as solvent.

4. A process as claimed in any one of claims 1 to 3 wherein the molar ratio of the compound of formula II: chloromethyl chloroformate: base used is 1:(2.1 to 2.6):(2.1 to 2.6).

5. A process as claimed in any one of claims 1 to 4 which comprises using pyridine as the base.

6. A process as claimed in any one of claims 1 to 5 which comprises carrying out the reaction at a temperature of between -30°C and +10°C.

7. A process as claimed in any one of claims 1 to 6 which comprises carrying out the reaction at about -5°C.

8. The use of the compound of formula (I) as defined in claim 1 in the preparation of 2,6-bis-(hydroxymethyl)pyridine carbamate esters.

9. A process for the preparation of 2,6-bis(hydroxymethyl)pyridine bis(N-methylcarbamate) which comprises reacting the compound of formula (I) as defined in claim 1 with methylamine.

## Patentansprüche

**Patentansprüche für folgende Vertragsstaaten : BE, CH, DE, FR, GB, GR, IT, LI, NL, SE**

1. 2,6-Bis(chloromethyloxy-carbonyloxy-methyl)pyridin der Formel (I):

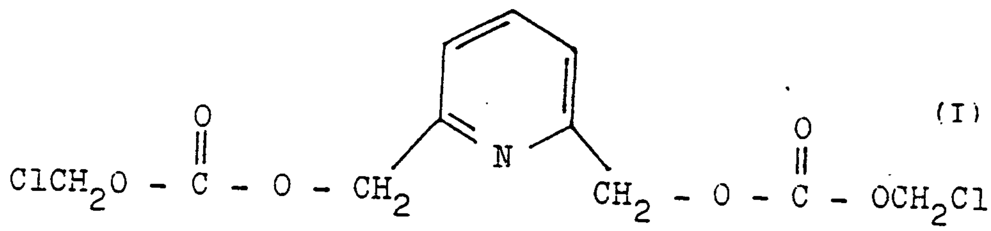

(I)

2. Verfahren zur Herstellung von 2,6-Bis(chloromethyloxy-carbonyloxy-methyl)pyridin der Formel (I):

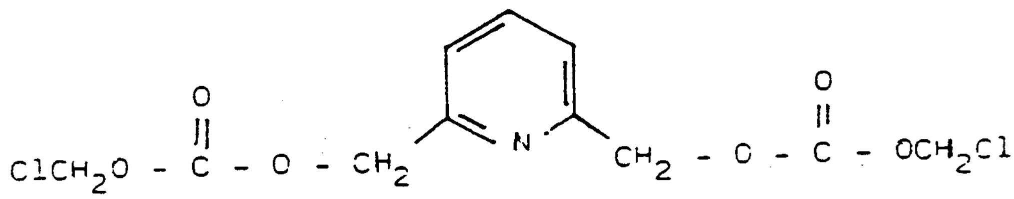

(I)

umfassend die Reaktion von 2,6-Bis(hydroxymethyl)-pyridin der Formel (II)

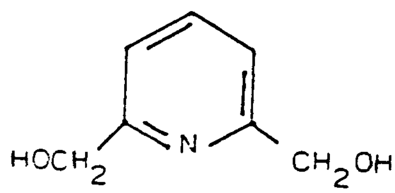

(II)

mit Chloromethylchloroformiat, wahlweise in der Gegenwart einer weiteren Base.

3. Verfahren nach Anspruch 2, dadurch **gekennzeichnet,** daß die Reaktion in einem polaren, aprotischen, mit Wasser nicht mischbaren, organischen Lösungsmittel durchgeführt wird.

4. Verfahren nach Anspruch 2 oder Anspruch 3, umfassend die Verwendung eines halogenierten Kohlenwasserstoffes als Lösungsmittel.

5. Verfahren nach einem der Ansprüche 2 bis 4, dadurch **gekennzeichnet,** daß das molare Verhältnis der Verbindung der Formel (II): Chloromethylchloroformiat: Base, die verwendet wird, 1:(2,1 bis 2,6): (2,1 bis 2,6) ist.

6. Verfahren nach einem der Ansprüche 2 bis 5, umfassend die Verwendung von Pyridin als Base.

7. Verfahren nach einem der Ansprüche 2 bis 6, dadurch **gekennzeichnet,** daß die Reaktion bei einer Temperatur zwischen -30 °C und +10 °C durchgeführt wird.

**8.** Verfahren nach einem der Ansprüche 2 bis 7, umfassend die Durchführung der Reaktion bei etwa -5 ° C.

**9.** Verwendung der Verbindung der Formal (I) nach Anspruch 1 für die Herstellung von 2,6-Bis-(hydroxymethyl)pyridincarbamatestern.

**10.** Verfahren zur Herstellung von 2,6-Bis(hydroxymethyl)pyridin- bis(N-methylcarbamat), umfassend die Reaktion der Verbindung der Formel (I) nach Anspruch 1 mit Methylamin.

**Patentansprüche für folgende Vertragsstaaten : AT, ES**

**1.** Verfahren zur Herstellung von 2,6-Bis(chloromethyloxy-carbonyloxy-methyl)pyridin der Formel (I):

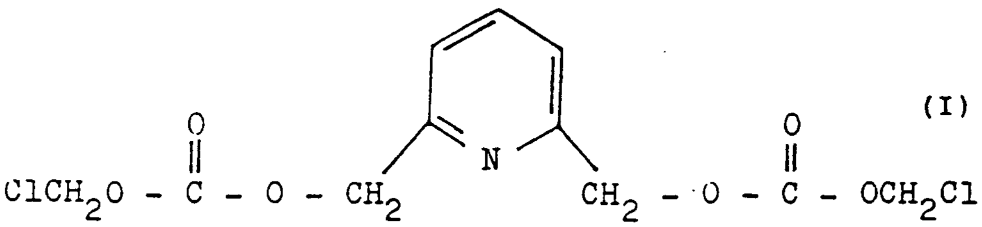

(I)

umfassend die Reaktion von 2,6-Bis(hydroxymethyl)-pyridin der Formel (II)

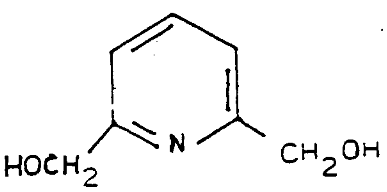

(II)

mit Chloromethylchloroformiat, wahlweise in der Gegenwart einer weiteren Base.

**2.** Verfahren nach Anspruch 1, dadurch **gekennzeichnet,** daß die Reaktion in einem polaren, aprotischen, mit Wasser nicht mischbaren, organischen Lösungsmittel durchgeführt wird.

**3.** Verfahren nach Anspruch 1 oder Anspruch 2, umfassend die Verwendung eines halogenierten Kohlenwasserstoffes als Lösungsmittel.

**4.** Verfahren nach einem der Ansprüche 1 bis 3, dadurch **gekennzeichnet,** daß das molare Verhältnis der Verbindung der Formel (II): Chloromethylchloroformiat: Base, die verwendet wird, 1:(2,1 bis 2,6): (2,1 bis 2,6) ist.

**5.** Verfahren nach einem der Ansprüche 1 bis 4, umfassend die Verwendung von Pyridin als Base.

**6.** Verfahren nach einem der Ansprüche 1 bis 5, dadurch **gekennzeichnet,** daß die Reaktion bei einer Temperatur zwischen -30 ° C und +10 ° C durchgeführt wird.

**7.** Verfahren nach einem der Ansprüche 1 bis 6, umfassend die Durchführung der Reaktion bei etwa -5 ° C.

**8.** Verwendung der Verbindung der Formel (I) wie in Anspruch 1 definiert zur Herstellung von 2,6-Bis-(hydroxymethyl)pyridincarbamatestern.

**9.** Verfahren zur Herstellung von 2,6-Bis(hydroxymethyl)pyridin- bis(N-methylcarbamat), umfassend die Reaktion der Verbindung der Formel (I) wie in Anspruch 1 definiert mit Methylamin.

**Revendications**

**Revendications pour les Etats contractants suivants : BE, CH, DE, FR, GB, GR, IT, LI, NL, SE**

**1.** 2,6-Bis(chlorométhyloxycarbonyloxyméthyl)-pyridine de formule (I) :

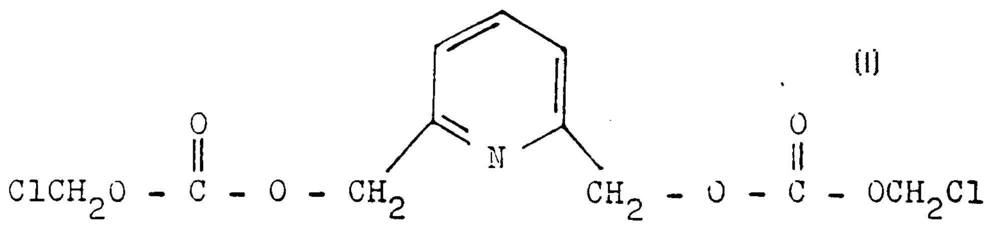

(I)

**2.** Procédé pour la préparation de la 2,6-bis-(chlorométhyloxycarbonyloxyméthyl)-pyridine de formule (I) :

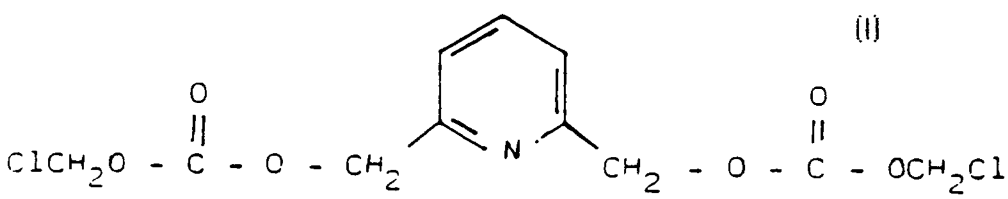

(I)

qui comprend la réaction de la 2,6-bis-(hydroxyméthyl)-pyridine de formule (I) :

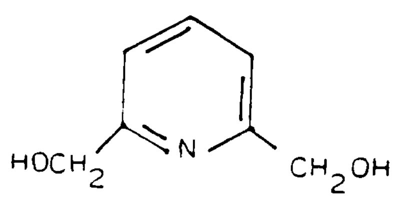

(II)

avec du chloroformiate de chlorométhyle, éventuellement en présence d'une base supplémentaire.

**3.** Procédé suivant la revendication 2, dans lequel la réaction est effectuée dans un solvant organique immiscible à l'eau, aprotique et polaire.

**4.** Procédé suivant la revendication 2 ou la revendication 3, qui comprend l'utilisation d'un hydrocarbure halogéné comme solvant.

**5.** Procédé suivant l'une quelconque des revendications 2 à 4, dans lequel le rapport molaire utilisé du composé de formule (II)/chloroformiate de chlorométhyle/base est de préférence de l'ordre de 1/(2,1 à 2,6)/(2,1 à 2,6).

**6.** Procédé suivant l'une quelconque des revendications 2 a 5, qui comprend l'utilisation de pyridine en tant que base.

**7.** Procédé suivant l'une quelconque des revendications 2 à 6, qui comprend la réalisation de la réaction a une température comprise entre -30 ° C et +10 ° C.

**8.** Procédé suivant l'une quelconque des revendications 2 à 7, qui comprend la réalisation de la réaction à environ -5° C.

**9.** Utilisation du composé de formule (I) suivant la revendication 1, dans la préparation d'esters carbamiques de 2,6-bis-(hydroxyméthyl)-pyridine.

**10.** Procédé pour la préparation du bis-(N-méthylcarbamate de 2,6-bis(hydroxyméthyl)-pyridine, qui comprend la réaction du composé de formule (I) suivant la revendication 1 avec de la méthylamine.

**Revendications pour les Etats contractants suivants : AT, ES**

**1.** Procédé pour la préparation de la 2,6-bis-(chlorométhyloxycarbonyloxyméthyl)-pyridine de formule (I) :

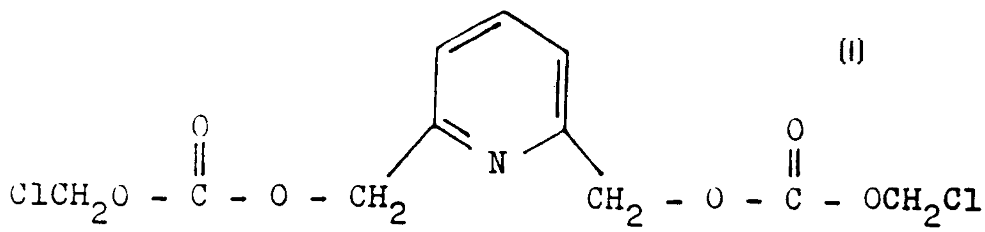

(I)

qui comprend la réaction de la 2,6-bis-(hydroxyméthyl)-pyridine de formule (II) :

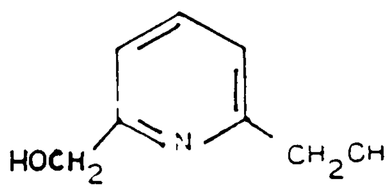

(II)

avec du chloroformiate de chlorométhyle, éventuellement en présence d'une base supplémentaire.

**2.** Procédé suivant la revendication 1, dans lequel la réaction est effectuée dans un solvant organique, immiscible à l'eau, aprotique et polaire.

**3.** Procédé suivant la revendication 1 ou la revendication 2 qui comprend l'utilisation d'un hydrocarbure halogéné comme solvant.

**4.** Procédé suivant l'une quelconque des revendications 1 à 3, dans lequel le rapport molaire utilisé du composé de formule (II)/chloroformate de chlorométhyle/base est de préférence de l'ordre de 1/(2,1 à 2,6)/(2,1 à 2,6).

**5.** Procédé suivant l'une quelconque des revendications 1 à 4, qui comprend l'utilisation de pyridine en tant que base.

**6.** Procédé suivant l'une quelconque des revendications 1 à 5 qui comprend la réalisation de la réaction a une température comprise entre -30° C et + 10° C.

**7.** Procédé suivant l'une quelconque des revendications 1 a 6, qui comprend la réalisation de la réaction a environ -5° C.

**8.** Utilisation du composé de formule (I) suivant la revendication 1, dans la préparation d'esters carbamiques de 2,6-bis-(hydroxyméthyl)-pyridine.

**9.** Procédé pour la préparation du bis-(N-méthylcarbamate de 2,6-bis(hydroxyméthyl)-pyridine, qui comprend la réaction du composé de formule (I) suivant la revendication 1, avec de la méthylamine.